# EUROPÄISCHE PATENTSCHRIFT

(11) **EP 3 590 435 B1**
(45) Veröffentlichungstag und Bekanntmachung des Hinweises auf die Patenterteilung: **10.03.2021**
(21) Anmeldenummer: 18181474.0
(22) Anmeldetag: 03.07.2018
(51) Int. Cl.: A61B 8/08, A61B 8/00

(54) **STEUERUNG VON ULTRASCHALLAUFNAHMEN**
CONTROL OF ULTRASONIC IMAGES
COMMANDE D'IMAGERIE ULTRASONORE

(43) Veröffentlichungstag der Anmeldung: 08.01.2020
(73) Patentinhaber: Siemens Healthcare GmbH, 91052 Erlangen (DE)
(72) Erfinder: Düppenbecker, Peter Michael, 91074 Herzogenaurach (DE); Radicke, Marcus, 90587 Veitsbronn (DE); Schmidt, Oliver, 91052 Erlangen (DE)

(56) Entgegenhaltungen:
- WO-A1-2006/035381
- DE-A1-102014 202 745
- US-A1- 2008 242 979
- US-A1- 2017 128 037
- US-A1- 2017 281 131

## Beschreibung

Die Erfindung betrifft ein Verfahren zur Steuerung von Ultraschallaufnahmen, eine Steuereinrichtung zur Steuerung einer Ultraschallanlage sowie eine Ultraschallanlage mit einer solchen Steuereinrichtung. Zudem betrifft die Erfindung eine Röntgenanlage mit einer Ultraschallanlage.

Medizinische Untersuchungen einer Brust, insbesondere die einer menschlichen weiblichen Brust, werden in der Regel durchgeführt, um bösartige Veränderungen des Brustgewebes gezielt und sicher feststellen bzw. diagnostizieren zu können. Eine gängige Methode ist die Mammographie, um solch eine Untersuchung durchführen zu können.

Bei einer mammographischen Untersuchung wird die Brust in einer Röntgenanlage bzw. der Mammographieanlage zwischen zwei übereinander ausgerichteten Kompressionsplatten positioniert und komprimiert. Wobei die unterste Kompressionsplatte als Lagerplatte der Brust dient. Daraufhin wird ein Röntgenbild der komprimierten Brust erzeugt. Dazu werden mittels einer Röntgenquelle Röntgenstrahlen ausgesandt. Diese werden beim Passieren der Brust in ihrer Intensität geschwächt und anschließend von einem Detektor erfasst.

Es soll in diesem Zusammenhang bereits hier darauf hingewiesen werden, dass sich die Begriffe "über" und "unter" bzw. "oben" und "unten" etc. im Rahmen der vorliegenden Erfindung im Folgenden ohne Beschränkung der Allgemeinheit auf die Position bzw. Ausrichtung einer zu untersuchenden Brust in einer Mammographieanlage beziehen. Dabei entspricht "nach oben" einer Richtung, die vom Detektor zur Röntgenquelle weist, und "nach unten" der entgegengesetzten Richtung.

Je nach Befund oder Gewebebeschaffenheit der zu untersuchenden Brust kann es von Vorteil sein, noch zusätzliche bzw. ergänzende Informationen zu dem zu untersuchenden Brustgewebe zu ermittelten. Daher werden zu den Röntgenaufnahmen oftmals noch Ultraschallaufnahmen der Brust aufgenommen, wobei eine Ultraschallsonde eines Ultraschallgeräts in der Regel zwischen der Röntgenquelle und dem Detektor angeordnet ist. Die Ultraschallsonde, der Detektor und die Kompressionsplatten sind in der Regel parallel zueinander auf unterschiedlichen Transversalebenen angeordnet. Die Transversalebenen erstrecken sich dabei im Wesentlichen senkrecht zu einer Ausbreitungsrichtung ausgesendeter Ultraschallwellen (wie noch erläutert wird).

Die Ultraschallsonde weist dabei eine Oberseite auf und eine dazu angeordnete Unterseite. Die Unterseite der Ultraschallsonde weist dabei in Richtung einer Oberseite einer zu untersuchenden Brust.
Bei einer Ultraschallaufnahme bzw. Sonographieaufnahme werden mittels der Ultraschallsonde Ultraschallwellen in Richtung eines zu untersuchenden Objekts, wie beispielsweise der Brust eines Patienten, ausgesendet. Die Ultraschallwellen werden an den unterschiedlichen Strukturen des zu untersuchenden Objekts gestreut, gebrochen und reflektiert. Die daraufhin zurückkommenden Ultraschallwellen werden von der Ultraschallsonde wieder empfangen und anschließend ausgewertet und umgewandelt, so dass ein Bild des Inneren des zu untersuchenden Objekts erzeugt werden kann. Diese bildliche Darstellung kann dabei sowohl 2-dimensional als auch 3- dimensional erfolgen.

In der Regel wird eine Ultraschallaufnahme bzw. ein Ultraschallscan automatisch und mit einem gesamten Aufnahmevolumen bzw. maximalen Aufnahmevolumen einer entsprechenden Ultraschallsonde getätigt. Es spielt dabei keine Rolle wie groß der tatsächlich zu untersuchende Bereich ist bzw. wie groß die zu untersuchende Brust ist. Daher ergibt sich aus dieser zusätzlichen Untersuchung der Brust ein relativ hoher Mehraufwand an Zeit, wodurch beispielsweise auch der klinische Workflow negativ beeinflusst wird.

Ein nächster Stand der Technik für die Erfindung ist aus US 2017/128037 bekannt. US 2017/128037 offenbart ein Verfahren zur Steuerung von Ultraschallaufnahmen aufweisend zumindest folgende Schritte:
- Ermittlung eines Ausdehnungsmaßes einer Brust und
- Regelung eines aktiven Bereichs einer Ultraschallsonde einer Ultraschallanlage in Abhängigkeit des ermittelten Ausdehnungsmaßes.

Es ist eine Aufgabe der vorliegenden Erfindung, die Untersuchungszeit bzw. Aufnahmezeit einer vollständigen, die gesamte Brust umfassenden Ultraschallaufnahme zu verkürzen.
Diese Aufgabe wird durch ein Verfahren gemäß Patentanspruch 1, durch eine Steuereinrichtung gemäß Patentanspruch 11, eine Ultraschallanlage gemäß Patentanspruch 12 sowie durch eine Röntgenanlage gemäß Patentanspruch 13 gelöst.
Bei dem erfindungsgemäßen Verfahren zur Steuerung von Ultraschallaufnahmen bzw. Sonographieaufnahmen wird ein Ausdehnungsmaß einer zu untersuchenden Brust, insbesondere einer menschlichen weiblichen Brust ermittelt. Das Ausdehnungsmaß beschreibt dabei eine Kontur bzw. einen 2D-Umriss der Brust. Das Ausdehnungsmaß beschreibt bei einer Draufsicht auf eine Brust bzw. in einer ersten Ebene bzw. in der Transversalebene die Kontur bzw. die Außenränder der Brust.
In Abhängigkeit des ermittelten Ausdehnungsmaßes der Brust wird daraufhin ein aktiver Bereich einer Ultraschallsonde bzw. eines Ultraschallkopfes einer Ultraschallanlage geregelt. Der aktive Bereich beschreibt dabei den Bereich der Ultraschallsonde, mit welchem Ultraschallwellen zur Erzeugung eines Ultraschallbildes der zu untersuchenden Brust eines Patienten ausgesendet und empfangen werden. Durch die Regelung bzw. die adaptive Einstellung des aktiven Bereichs, entsprechend dem zuvor ermittelten Ausdehnungsmaß, wird nun also nur mittels des Bereichs der Ultraschallsonde eine Aufnahme getätigt, unter dem sich die zu untersuchende Brust befindet. Es wird also nicht mehr, wie dies zuvor der Fall war, ein gesamtes Volumen unterhalb der Ultraschallsonde gescannt. Vielmehr erfolgt der Scan erfindungsgemäß abhängig davon, ob sich unterhalb der Ultraschallsonde noch Bereiche der zu untersuchenden Brust befinden oder nicht. Mittels der Erfindung kann so die Untersuchungszeit enorm verkürzt werden, was unter anderem auch einen positiven Effekt auf den medizinischen Workflow und den Komfort des Patienten hat. Bisher wurde, wie bereits erwähnt, unabhängig von einer Größe einer zu untersuchenden Brust ein maximales Volumen (ca. 30 x 18 x 10 cm³) unterhalb einer Ultraschallsonde gescannt. Wird nun mit dem erfindungsgemäßen Verfahren beispielsweise eine durchschnittlich große Brust gescannt, welche ca. ein Volumen von 15x 9x 5 cm³ im komprimierten Zustand aufweist, so können Zeitersparnisse ca. von einem Faktor 8 erfolgen.

Zudem kann durch den angepassten Aufnahmebereich bzw. Scanbereich auch das durch den Ultraschallscan erhaltende Datenvolumen stark reduziert werden.

Eine entsprechende erfindungsgemäße Steuereinrichtung zur Steuerung einer Ultraschallanlage weist zumindest eine Analyseeinheit zur Bestimmung eines Ausdehnungsmaßes einer Brust sowie eine Regeleinheit zur Regelung eines aktiven Bereichs einer Ultraschallsonde einer Ultraschallanlage auf.

Soll mittels einer bereits bestehenden Ultraschallanlage die Untersuchungszeit bzw. die Aufnahmezeit einer Ultraschallaufnahme einer Brust verkürzt werden, so kann die Ultraschallanlage um eine oben genannte erfindungsgemäße Steuereinrichtung ergänzt werden bzw. die Steuereinrichtung, wie nachfolgend beschrieben, nachgerüstet werden. Eine Ultraschallanlage kann aber auch direkt während der Fertigung mit einer erfindungsgemäßen Steuereinrichtung ausgestaltet werden, um so die Aufnahmezeit einer zu untersuchenden Brust zu verkürzen.

Um neben den Ultraschallaufnahmen noch weitere Informationen zum Brustgewebe einer zu untersuchenden Brust zu erlangen, können auch Röntgenaufnahmen der Brust durchgeführt werden. Dazu kann die erfindungsgemäße Ultraschallanlage in einer Röntgenanlage integriert sein.

Bei der Röntgenanlage handelt es sich bevorzugt um eine Mammographieanlage und/oder Tomosyntheseanlage. Diese Systeme dienen vor allem, wie bereits erläutert, zur Früherkennung von Tumoren in einer menschlichen Brust.

Ein Großteil der zuvor genannten Komponenten der Ultraschallanlage können ganz oder teilweise in Form von Softwaremodulen in einem Prozessor einer entsprechenden Steuereinrichtung realisiert werden. Eine weitgehend softwaremäßige Realisierung hat den Vorteil, dass auch schon bisher verwendete Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt gelöst, welches direkt in eine Speichereinrichtung einer Steuereinrichtung einer Ultraschallanlage ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation und / oder zusätzliche Komponenten, auch Hardware-Komponenten, wie z. B. Hardware-Schlüssel (Dongles etc.) zur Nutzung der Software, umfassen.

Zum Transport zur Steuereinrichtung und / oder zur Speicherung an oder in der Steuereinrichtung kann ein computerlesbares Medium, beispielsweise ein Memory Stick, eine Festplatte oder ein sonstiger transportabler oder fest eingebauter Datenträger dienen, auf welchem die von einer Rechnereinheit der Steuereinrichtung einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Die Ultraschallsonde der erfindungsgemäßen Ultraschallanlage kann unterschiedlich ausgebildet sein. So kann sie z. B. als konvexe, sektorförmige Sonde ausgebildet sein. Vorzugsweise handelt es sich bei der Ultraschallsonde der erfindungsgemäßen Ultraschallanlage aber um eine lineare oder flächige Ultraschallsonde. Die lineare Ultraschallsonde ist bevorzugt in ihrer Haupterstreckungsrichtung, d.h. in Richtung ihrer länglichen Ausdehnung, in einer ersten Richtung, z. B. von anterior nach posterior, medio-lateral oder medio-lateraloblique angeordnet.

Das Ausdehnungsmaß der Brust kann beispielsweise mittels eines Messschiebers oder aber auch durch Kennzeichnung auf den Kompressionsplatten einer Röntgenanlage, sofern sich die zu untersuchende Brust zwischen den Kompressionsplatten befindet, ermittelt werden.

Das Ausdehnungsmaß kann bevorzugt auch mittels einer Aufnahme eines optischen Bildes beispielsweise durch eine Kamera- bzw. Fotoaufnahme der Brust ermittelt werden.

Besonders bevorzugt wird das Ausdehnungsmaß der Brust aber mittels einer Röntgenaufnahme ermittelt. Diese kann z. B. mittels der erfindungsgemäßen Röntgenanlage akquiriert worden sein.

Das Ausdehnungsmaß kann dabei z. B. mittels einer Kantendetektion, einer Schwellwertanalyse oder der dergleichen ermittelt werden.

Ist eine zu untersuchende Brust in einer Röntgenanlage angeordnet, kann vorzugsweise neben dem Ausdehnungsmaß der zu untersuchenden Brust auch eine Position auf einem Detektor der Röntgenanlage ermittelt werden. Die Positionsbestimmung erfolgt vorzugsweise ebenfalls mittels einer Röntgenaufnahme. Durch die Ermittlung der Position der zu untersuchenden Brust kann ebenso eine Ultraschallaufnahme und gegebenenfalls eine Röntgenaufnahme noch patientengerechter bzw. individueller eingestellt werden.

Nachdem das Ausdehnungsmaß ermittelt wurde kann, wie bereits beschrieben, der aktive Bereich der Ultraschallsonde geregelt bzw. eingestellt werden. Der aktive Bereich der Ultraschallsonde erstreckt sich dabei in einer Transversalebene bis zu einem Randbereich bzw. einer Randgrenze der Brust. Hierbei gibt es verschiedene Ausgestaltungsmöglichkeiten.

In einer bevorzugten Ausgestaltung des aktiven Bereichs erstreckt sich der aktive Bereich aber in der ersten Richtung der Transversalebene, bevorzugt von anterior nach posterior. Besonders bevorzugt erstreckt sich der aktive Bereich von einem Bereich des Brustansatzes, also z. B. vom Musculus Pectoralis, bis zu einem gegenüberliegenden Endbereich der Brust, also z. B. einer Mamille (bei mittiger Anordnung der Ultraschallsonde).

Alternativ oder zusätzlich erstreckt sich der aktive Bereich in einer zweiten Richtung der Transversalebene, welche senkrecht zur ersten Richtung ausgerichtet ist, bevorzugt von medial nach lateral, besonders bevorzugt von einem Randbereich der Brust zu einem in dieser Richtung gegenüberliegenden Randbereich der Brust.

Dabei sind beide Richtungen grundsätzlich gleichwertig. Insbesondere erfolgt bei einer flächigen Ausgestaltung des Schallkopfes bevorzugt auch die Regelung des aktiven Bereiches in beiden Richtungen der Transversalebene.

Der aktive Bereich der Ultraschallsonde wird dabei bevorzugt durch eine Auswahl an aktiven Elementen bzw. Piezoelementen der Ultraschallsonde eingestellt. Bei diesen Elementen handelt es sich vorzugsweise um linear nebeneinander liegende Sende- und Empfangselemente, welche entsprechend Ultraschallwellen aussenden und zurückkommende, reflektierte Ultraschallwellen empfangen. Ein erstes aktives Element liegt vorzugsweise oberhalb des Brustgewebes der zu untersuchenden Brust und benachbart zum Pectoralis. Ein letztes aktives Element liegt vorzugsweise oberhalb des Brustgewebes und benachbart zu einer dem Pectoralis gegenüberliegenden Randgrenze der Brust an.

In Abhängigkeit des ermittelten Ausdehnungsmaßes kann vorzugsweise zudem auch ein Verfahrbereich der Ultraschallsonde eingestellt werden. Die Ultraschallsonde verfährt dabei bevorzugt in einer Transversalebene und wie bereits erwähnt, besonders bevorzugt oberhalb der Brust.

Der Verfahrbereich der Ultraschallsonde erstreckt sich bevorzugt senkrecht zu der Richtung der Transversalebene, in der sich der aktive Bereich der Ultraschallsonde erstreckt.

Erstreckt sich beispielsweise der aktive Bereich, wie oben beschrieben, in der ersten Richtung der Transversalebene, so erstreckt sich der Verfahrbereich in der zweiten Richtung der Transversalebene. Besonders bevorzugt erstreckt sich der Verfahrbereich von medial nach lateral. Ganz besonders bevorzugt erstreckt er sich von einem Randbereich der Brust zu einem in dieser Richtung gegenüberliegenden Randbereich der Brust. Der Verfahrbereich ist dabei durch die Stelle gekennzeichnet, an der zwischen einer Randgrenze zu gegenüberliegenden Randgrenze der Brust ein maximaler Abstand besteht.

Verfährt nun die Ultraschallsonde, vorzugsweise eine motorisierte Ultraschallsonde, entlang des Verfahrbereichs, so ändert sich im Fahrtverlauf, aufgrund der Geometrie der Brust, auch die Größe des Bereichs, unter dem sich die zu untersuchende Brust befindet. Daher wird der aktive Bereich der Ultraschallsonde in Abhängigkeit des Ausdehnungsmaßes vorzugsweise während des Verfahrens der Ultraschallsonde entlang des Verfahrbereichs adaptiv angepasst bzw. geregelt.

Auch eine Verfahrgeschwindigkeit der Ultraschallsonde kann bevorzugt in Abhängigkeit des ermittelten Ausdehnungsmaßes geregelt werden. Dabei verfährt die Ultraschallsonde bevorzugt entlang des Verfahrbereichs.

Durch die individuelle Einstellung des Verfahrbereichs und der Verfahrgeschwindigkeit der Ultraschallsonde kann sich die Untersuchungszeit entsprechend reduzieren.

Zusätzlich zum Ausdehnungsmaß der Brust kann auch eine Dicke, vorzugsweise eine Kompressionsdicke der Brust, ermittelt werden. Diese kann beispielsweise mithilfe eines Abstands der Kompressionsplatten der Röntgenanlage ermittelt werden. Die Kompressionsdicken einer menschlichen weiblichen Brust liegen dabei durchschnittlich zwischen ca. 10 cm und ca. 2 cm. Daraus resultiert ein maximaler Patienten-Individualfaktor von > 4.

Auf Basis der ermittelten Dicken wird dann bevorzugt eine Zeit bzw. eine Wartezeit zwischen Aussendung eines Ultraschallsendepulses und Empfang eines Ultraschallempfangspulses angepasst bzw. geregelt. Auch die Verfahrgeschwindigkeit der Ultraschallsonde bzw. eines Motors der Ultraschallsonde kann gegebenenfalls auf Basis der Dicke bzw. Kompressionsdicke der Brust erneut angepasst werden. Auch hierdurch kann sich die Untersuchungszeit stark verkürzen.

In Abhängigkeit der ermittelten Dicke kann ebenfalls die Frequenz des ausgesendeten Ultraschallsendepulses patientenindividuell eingestellt werden.

Dabei wird bei einer relativ geringen Eindringtiefe eine Ultraschallwelle mit einer Frequenz von bevorzugt mindestens 10 MHz, besonders bevorzugt von mindestens 14 MHz ausgesendet. Bei einer relativ hohen Eindringtiefe wird eine Ultraschallwelle mit einer Frequenz von bevorzugt maximal 8 MHz, besonders bevorzugt maximal 6 MHz ausgesendet. Hierdurch kommt es zu einer weiteren Optimierung der Bildqualität der Ultraschallaufnahme der Brust.

Um verschieden tiefe Ebenen der Brust mit einer hohen Auflösung abbilden zu können, werden bevorzugt mehrere Ultraschallsendepulse bei einer im Wesentlichen gleichbleibenden Position der Ultraschallsonde oberhalb einer zu untersuchenden Brust ausgesendet. Unter einer "im Wesentlichen" gleichbleibenden Position wird in diesem Zusammenhang eine Position der Ultraschallsonde verstanden, in welcher Bildgebungsdaten schnell im Verhältnis zur akquiriert Verfahrgeschwindigkeit werden. D. h., die Ultraschallsonde bewegt sich dementsprechend verglichen mit der Geschwindigkeit des Akquisitionsprozesses nur relativ langsam entlang des Verfahrbereichs.

Die Ultraschallwellen, welche von der Ultraschalllsonde bei einer im Wesentlichen gleichbleibenden Position ausgesendet werden, unterscheiden sich dabei vorzugsweise in ihrer Frequenz voneinander. Durch die Einstellung der Frequenz kann ein axiales Auflösungsvermögen, d.h. das Auflösungsvermögen in Ausbreitungsrichtung der Ultraschallwellen, beeinflusst und optimiert werden. Das axiale Auflösungsvermögen ist durch die Länge eines Ultraschallimpulses bestimmt und entspricht dabei oftmals einer oder mehrerer Ultraschallwellenlängen. Je höher die Ultraschallsendefrequenz ist bzw. je kürzer die damit verbundene Wellenlänge ist, desto höher ist die axiale Auflösung.

Die Ultraschallwellen können sich aber auch vorzugsweise durch eine unterschiedliche Fokussierung voneinander unterscheiden. Durch die Einstellung der Fokussierung der Ultraschallwellen bzw. einer Fokuszone wird ein laterales Auflösungsvermögen, welches sich senkrecht zur Ausbreitungsrichtung der Ultraschallwellen erstreckt, beeinflusst. Je schmaler das Ultraschallfeld ist bzw. je dichter die Schallwellen in der Fokuszone liegen, desto größer ist die Detailerkennbarkeit bzw. die laterale Auflösung. Durch die zeitlich versetzte Ansteuerung der Ultraschallelemente bzw. Piezoelemente kann so die Fokuszone, d. h. der Bereich mit der höchsten lateralen Auflösung, patientenindividuell in unterschiedliche Tiefen der Brust eingestellt bzw. verfahren werden (1,5-D-Schallkopf).
Die durch die mehreren ausgesendeten Ultraschallsendepulse entstehenden Ultraschallbilder können dann anschließend bevorzugt zu einem Ultraschallbild zusammengefügt werden.

Mittels der Erfindung können also automatisiert geregelte und optimal abgestimmte Ultraschallparameter, wie beispielsweise, der Verfahrbereich, die Verfahrgeschwindigkeit, die Frequenz, die Wartezeit zwischen Ultraschallempfangssendepuls und Ultraschallsendepuls etc. für jeden Patienten individuell eingestellt werden. Die Einstellungen werden dabei je nach den Anforderungen der spezifischen Anwendung und in Abhängigkeit von den Abmessungen der Brust gewählt.

Bei der Aufnahme einer ganzen Brust werden also erst Ultraschallparameter, wie beispielsweise der Verfahrbereich, die Verfahrgeschwindigkeit etc. für einen Ultraschallscan ermittelt. Diese Einstellungsparameter werden dann an ein Steuerprotokoll bzw. Untersuchungsprotokoll übergeben, das die zeitlich koordinierte Abfolge und Durchführung eines Ultraschallscans einer ganzen Brust steuert.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich.

Die Lage bzw. Ausrichtung der dargestellten Komponenten bezieht sich hier auf eine sitzende bzw. stehende Position eines Patienten und eine kranial-kaudale Ausrichtung des Strahlengangs der Mammographieanlage. Jedoch sind grundsätzlich auch andere Positionen bzw. Ausrichtungen ebenfalls möglich und von der Erfindung umfasst.

Es zeigen:
Figur 1 schematisch eine Draufsicht auf eine menschliche Brust und eine über/oberhalb der Brust angeordnete Ultraschallsonde eines Ausführungsbeispiels einer erfindungsgemäßen Ultraschallanlage,
Figur 2 schematisch eine Seitenansicht der Brust und der Ultraschallsonde aus Figur 1,
Figur 3 schematisch eine Unteransicht der Ultraschallsonde aus FIG 1,
Figur 4 schematisch eine seitliche Schnittansicht eines Ausführungsbeispiels einer erfindungsgemäßen Röntgenanlage, in der ein Ausführungsbeispiel einer erfindungsgemäßen Ultraschallanlage mit einer Ultraschallsonde integriert ist, wobei ein aktiver Bereich der Ultraschallsonde passend zu einem Ausdehnungsmaß einer ersten zu untersuchenden Brust geregelt ist,
Figur 5 die Röntgenanlage und Ultraschallanlage aus FIG 4 in einer ähnlichen Ansicht wie in FIG 4, wobei ein aktiver Bereich der Ultraschallsonde passend zu einem Ausdehnungsmaß einer zweiten zu untersuchenden Brust geregelt ist,
Figur 6 eine Frontalansicht der Röntgenanlage und Ultraschallanlage aus FIG 4, wobei der Verfahrbereich und die Fokuszone der Ultraschallsonde passend zu einem Ausdehnungsmaß und einer Kompressionsdicke einer dritten zu untersuchenden Brust geregelt ist,
Figur 7 die Röntgenanlage und Ultraschallanlage aus FIG 4 in einer ähnlichen Ansicht wie in FIG 6, wobei der Verfahrbereich und die Fokuszone der Ultraschallsonde passend zu einem Ausdehnungsmaß und einer Kompressionsdicke einer vierten zu untersuchenden Brust geregelt ist und
Figur 8 ein Blockdiagramm für ein Ausführungsbeispiel des erfindungsgemäßen Verfahrens.

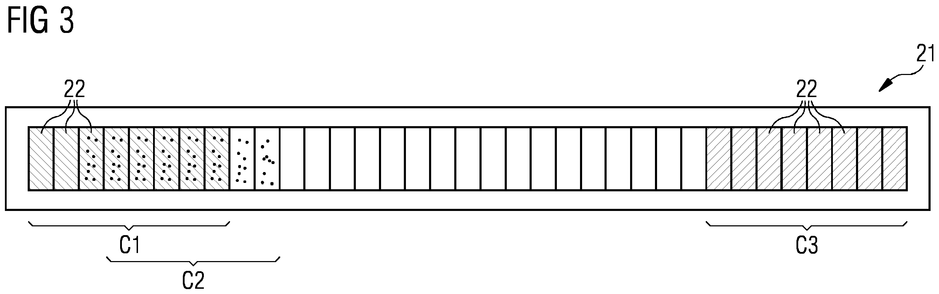

FIG 3 zeigt beispielhaft die Unteransicht einer Ultraschallsonde 21 einer erfindungsgemäßen Ultraschallanalage 20. Bei der Ultraschallsonde 21 handelt es sich hier um eine lineare Ultraschallsonde 21 bzw. Ultraschallkopf 21. In der Ultraschallsonde 21 sind einzelne Elemente 22 bzw. Piezoelemente 22 nebeneinander angeordnet. Die Piezoelemente 22 dienen dazu Ultraschallwellen auszusenden und zu empfangen. Es handelt sich also bei den Elementen 22 um einzelne nebeneinander angeordnete Sendeelemente und Empfangselemente.

Die Piezoelemente 22 sind in einzelne Gruppen C1, C2, C3 bzw. Cluster C1, C2, C3 unterteilt. Jeder Cluster C1, C2, C3 enthält dabei in der Regel ca. 192 Piezoelemente. Insgesamt weist eine Ultraschallsonde 21 ca. 1.000 bis ca. 1.200 Piezoelemente 22 auf. Zur besseren Darstellung wurde die Anzahl der Piezoelemente 22 in der FIG 3 auf 35 Elemente 22 begrenzt. In jedem Cluster C1, C2, C3 sind hier deshalb nur 8 Piezoelemente 22 angeordnet. Wird nun eine Ultraschallaufnahme durchgeführt, so werden alle Elemente 22 eines ersten Clusters C1 (hier mittels einer links oben nach rechts unten verlaufenden Schraffur gekennzeichnet) aktiviert. D. h., dass die Elemente 22 des ersten Clusters C1 entsprechend einen Ultraschallsende bzw. einen Ultraschallempfangspuls zur Bildgebung eines ersten Bereichs ausstrahlen bzw. empfangen. Daraufhin werden alle Elemente 22 eines zweiten Clusters C2 (hier mittels Punkten gekennzeichnet) zur Bildgebung eines zweiten Bereichs aktiviert. Fortlaufend werden nach dem Stand der Technik analog alle Elemente 22 der nebenstehenden Cluster aktiviert, bis ein letzter Bereich C3 (hier mittels einer von links unten nach rechts oben verlaufenden Schraffur gekennzeichnet) zur Bildgebung eines letzten Bereichs aktiviert wird. Die Aktivierung der Elemente 22 aller Cluster C1, C2, C3 und eine entsprechende Bildaufnahme erfolgen dabei nach dem Stand der Technik unabhängig von einer Größe der zu untersuchenden Brust. D. h. es werden unter Umständen auch Bildaufnahmen getätigt, wenn sich kein Brustgewebe mehr unter der Ultraschallsonde 21 befindet. Mittels der Erfindung können nun aber die aktiven Elemente 22 der Ultraschallsonde 21 patientenindividuell angewählt bzw. gesteuert werden, so dass unter Umständen nicht mehr mit allen Clustern C1, C2, C3 Bildaufnahmen durchgeführt werden müssen.

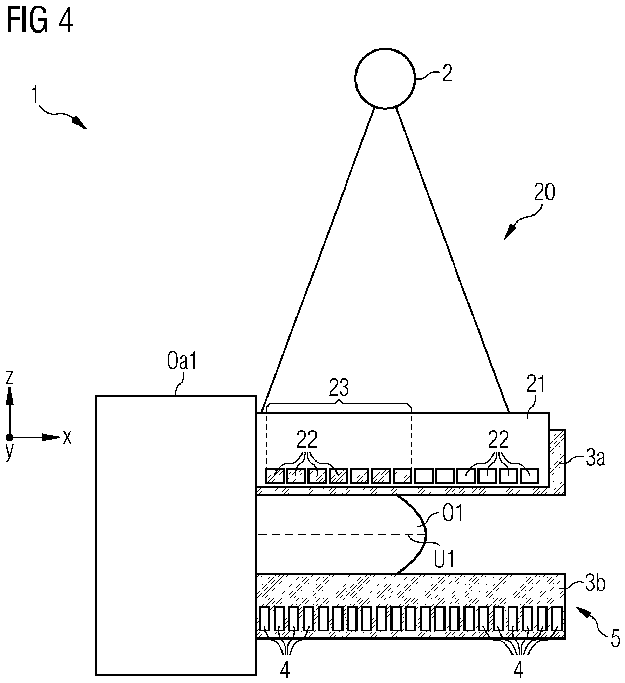

In FIG 4 ist dazu beispielhaft eine seitliche Schnittansicht einer Brust O1 eines Patienten in einer erfindungsgemäßen Röntgenanlage 1 gezeigt. Zur besseren Orientierung ist hier schematisch auch der an die Brust O1 angrenzende Pectoralis Oa1 dargestellt.

Zur besseren Orientierung der Lage bzw. Ausrichtung der Röntgenanlage und der darin befindlichen Brust, ist in den FIG 4 bis 8 ein Koordinatensystem eingezeichnet. Die x-Achse entspricht hierbei der Sagittalachse eines Patienten und ist anterior-posterier ausgerichtet. Die y-Achse ist hier die Querachse bzw. Transversalachse eines Patienten und ist medial-lateral ausgerichtet. Die z-Achse entspricht hier der Längsachse bzw. Longitudinalachse eines Patienten und ist kranial-kaudal ausgerichtet

Die erfindungsgemäße Röntgenanlage 1 weist hier eine verfahrbare motorisierte Röntgenquelle 2 und einen unterhalb der Röntgenquelle 2 liegenden Röntgendetektor 5 auf. Der Röntgendetektor 5 registriert die ankommenden Röntgenstrahlen und wandelt sie in messtechnisch nutzbare Größen um. Der Detektor 5 umfasst dazu mehrere in einer Ebene nebeneinander angeordnete Detektorpixel 4. Auf bzw. in dem Detektor 5 integriert befindet sich eine untere Kompressionsplatte 3b bzw. Auflageplatte 3b, auf der die zu untersuchende Brust O1 aufliegt. Mittels einer oberen Kompressionsplatte 3a wird die aufgelegte Brust O1 komprimiert.

In der FIG 4 wird exemplarisch von einer Mammographieanlage 1 als Röntgenanlage 1 ausgegangen, um Bilder des Inneren einer Brust bzw. des Untersuchungsobjekts, zu erhalten. Die Erfindung ist aber nicht hierauf beschränkt.

In der Röntgenanlage 1 ist ein Ausführungsbeispiel einer erfindungsgemäßen Ultraschallanlage 20 integriert, welche eine Ultraschallsonde 21, wie die aus Figur 3, aufweist. Die Ultraschallsonde 21 ist hier oberhalb der Brust O1 angeordnet und verläuft entlang einer Mittelachse der Brust O1 (hier nicht eingezeichnet in x-Richtung). In der Schnittansicht der Ultraschallsonde 21 sind auch hier die einzelnen Elemente 22 der Ultraschallsonde 21 zu erkennen.

Der Röntgendetektor 5, die obere Kompressionsplatte 3a, die untere Kompressionsplatte 3b und die Ultraschallsonde 21 sind hierbei parallel zueinander auf unterschiedlichen Transversalebenen angeordnet.

In Schwarz ist in FIG 4 ein aktiver Bereich 23 der Ultraschallsonde 21 gekennzeichnet, in welchem die Elemente 22, wie bereits erläutert, Ultraschallsendepulse aussenden und Ultraschallempfangspulse empfangen. Es ist zu erkennen, dass nur die Elemente 22 aktiv sind, unter welchen sich Gewebe der zu untersuchenden Brust O1 befindet. Um den aktiven Bereich 23 einzustellen, wird ein Ausdehnungsmaß U1 der Brust O1 ermittelt. Dieses Ausdehnungsmaß U1 wird hier mittels einer Röntgenaufnahme durch die Röntgenanlage 1 ermittelt.

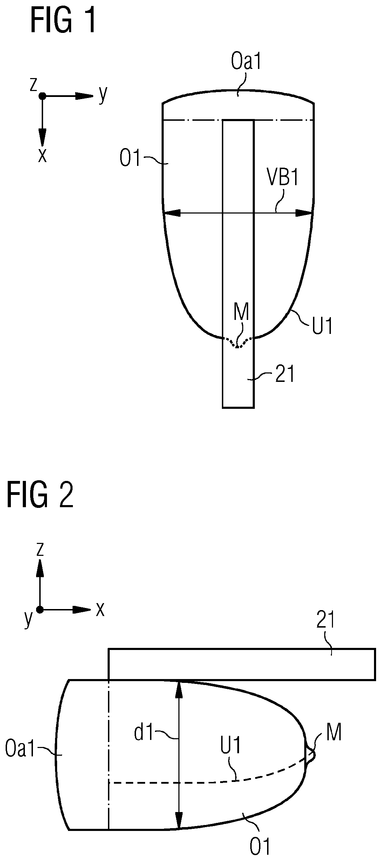

Das Ausdehnungsmaß U1 beschreibt dabei die Außenkontur der Brust O1 die bei einer Draufsicht auf die Brust O1 erkennbar ist (siehe hierzu FIG 1). Wurde das Ausdehnungsmaß U1 ermittelt so wird mittels einer Steuereinrichtung (hier nicht eingezeichnet) der Ultraschallanlage 20 der aktive Bereich 23 der Ultraschallsonde 21 entsprechend geregelt.

Weist eine zu untersuchende Brust eines Patienten ein anderes Ausdehnungsmaß auf, so wird der aktive Bereich 23 der Ultraschallsonde 21 entsprechend patientenindividuell angepasst.

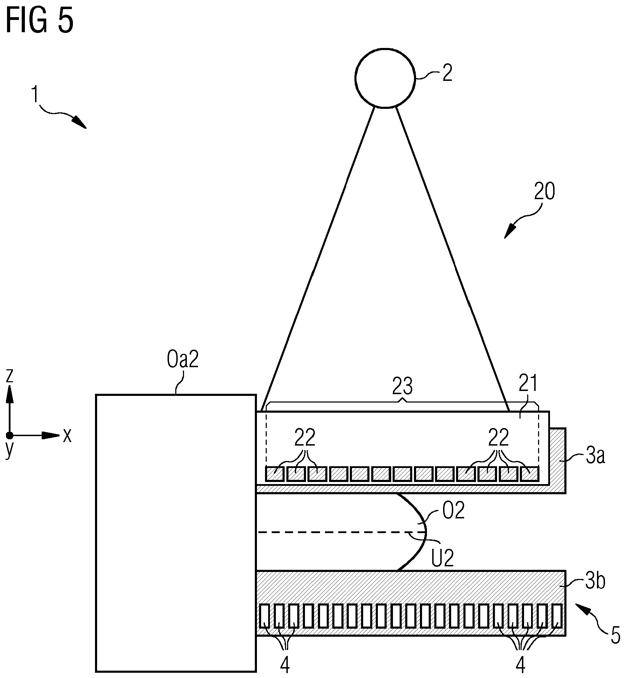

In FIG 5 ist beispielhaft eine zweite Brust O2 mit einem Ausdehnungsmaß U2 und dem angrenzenden Pectoralis Oa2 in der Röntgenanlage 1 aus FIG 4 dargestellt. Die zweite Brust O2 ist dabei im Wesentlichen so in der Röntgenanlage 1 angeordnet wie die erste Brust O1 aus FIG 4.

Das Ausdehnungsmaß U2 der zweiten Brust O2 erstreckt sich dabei in x-Richtung, von der Pectoralis Oa2 bis zu Mamille M (hier nicht eingezeichnet) über den gesamten Bereich der Ultraschallsonde 21, in welchem sich die einzelnen Elemente 22 befinden. Aus diesem Grund sind alle Elemente 22 der Ultraschallsonde 21 aktiv und tragen zur Bildgebung bei.

Damit nicht nur das Brustgewebe einer Brust in x-Richtung, also von anterior nach posterior aufgenommen werden kann, sondern auch in y-Richtung von medial nach lateral, kann die Ultraschallsonde 21 verfahren werden. Um auch hier die Untersuchungszeiten möglichst gering zu halten, kann ein Verfahrbereich VB1 (in FIG 1 gezeigt) individuell an einen Patienten angepasst werden

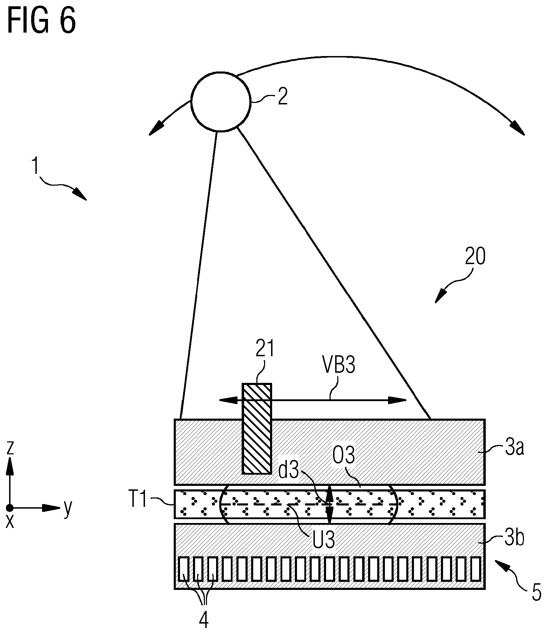

Die FIG 6 zeigt dazu beispielhaft die Frontansicht der Röntgenanlage 1 aus FIG 4, in der eine dritte Brust O3 eines weiteren Patienten untersucht wird. In Abhängigkeit eines zuvor ermittelten Ausdehnungsmaßes U3 der dritten Brust O3 verfährt nun die Ultraschallsonde 21 in einem Verfahrbereich VB3 in y-Richtung, also von medial von einem Randbereich bzw. Randgrenze der Brust nach lateral zu einer gegenüberliegenden Randseite bzw. Randbereich der Brust (siehe hierzu FIG 1). Die Ultraschallsonde 21 verfährt also auch in dieser Richtung nur in einem Bereich, in welchem sich Brustgewebe unter der Ultraschallsonde 21 befindet.

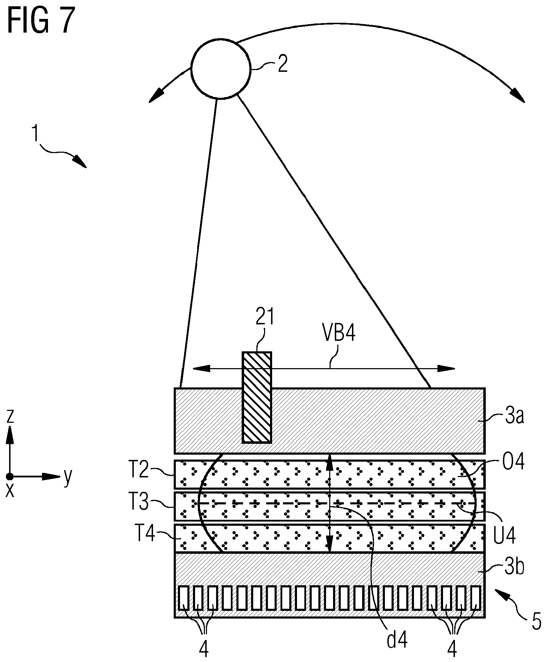

In FIG 7 ist im Vergleich dazu beispielhaft ein Verfahrbereich VB4 der Ultraschallsonde 21 aus FIG 4 gezeigt, der einem Ausdehnungsmaß U4 einer vierten Brust O4entspricht. Da das Ausdehnungsmaß U4 der vierten Brust O4 größer ist als das Ausdehnungsmaß U3 der dritten Brust O3, ist auch der Verfahrbereich VB2 der Ultraschallsonde 21 größer für die vierte zu untersuchende Brust O4.

Der maximale Verfahrbereich VB₁, VB3, VB4 wird dabei durch die breiteste Stelle einer Brust in y-Richtung bzw. von medial nach lateral definiert

Mittels der Kompressionsplatten 3a, 3b der Röntgenanlage 1 wird hier jeweils für eine zu untersuchende Brust O1, O2, O3, O4 eine Dicke bzw. eine Kompressionsdicke d1, d3, d4 ermittelt. Die Kompressionsdicke d1 für die erste zu untersuchende Brust O1 ist exemplarisch in der FIG 2 eingezeichnet (die Kompressionsplatten 3a, 3b wurden zur besseren Übersicht hier nicht dargestellt). Anhand der ermittelten Kompressionsdicke d1 der zu untersuchenden Brust O1, kann dann individuell eine maximal notwendige Wartezeit zwischen Ultraschallsendepuls und Ultraschallempfangspuls eingestellt werden. Hierdurch wird die Gesamtuntersuchungszeit der Brust O1 im Vergleich zu konventionellen Ultraschallaufnahmen verkürzt.

Anhand der ermittelten Kompressionsdicke d1, d3, d4 kann eine zu untersuchende Brust O1, O2, O3, O4 in verschiedene Ultraschallbildgebungszonen T1, T2, T3, T4 unterteilt werden.

Die vierte zu untersuchende Brust O4 aus FIG 7 ist in drei in z-Richtung übereinanderliegende, gleich dicke Ultraschallbildgebungszonen T2, T3, T4 unterteilt. Für jede Ultraschallbildgebungszone T2, T3, T4 kann anhand der ermittelten Kompressionsdicke d4 eine optimale Frequenz eingestellt werden, mit der die Ultraschallwellen ausgesendet werden. Zudem kann auch die Fokussierung der Ultraschallwellen an die jeweilige Bildgebungszone T2, T3, T4 angepasst werden.

Hierdurch wird ermöglicht, dass die verschieden tiefen Bildgebungszonen T2, T3, T4 mit einer besonders hohen Auflösung abgebildet werden können.

Die dritte zu untersuchende Brust O3 aus FIG 6 hat eine geringere Kompressionsdicke d3 als die vierte zu untersuchende Brust O4 aus FIG 7. Die dritte Brust O3 weist aufgrund ihrer Kompressionsdicke d3 daher nur eine Bildgebungszone T1 auf.

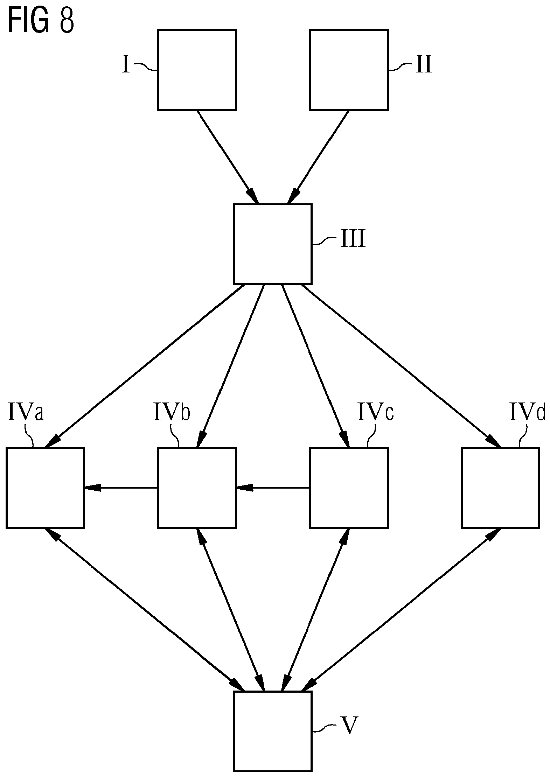

In FIG 8 ist ein Blockdiagramm gezeigt, mit dem ein Verfahren gemäß einem Ausführungsbeispiel der Erfindung veranschaulicht wird.

In einem ersten vorbereitenden Verfahrensschritt I wird eine zu untersuchende Brust komprimiert (was sie auch während der gesamten Ultraschallaufnahme bleibt).

Daraufhin wird die Dicke d1, d3, d4 bzw. die Kompressionsdicke d1, d3, d4 der zu untersuchenden Brust O1, O2, O3, O4 ermittelt, welche in der Röntgenanlage 1 angeordnet ist. Die Kompressionsdicke d1, d3, d4 wird hier mittels des Abstands zwischen den Kompressionsplatten 3a, 3b der Röntgenanlage 1 bestimmt.

In einem Verfahrensschritt II wird ein Ausdehnungsmaß U1, U2, U3, U4 der Brust O1, O2, O3, O4 ermittelt. Dies kann beispielsweise mittels eines Fotos, eines Messschiebers oder bevorzugt mittels einer Röntgenaufnahme ermittelt werden, die zuvor mit der Röntgenanlage 1 akquiriert wurde. Die Verfahrensschritte I und II können zeitgleich erfolgen. Der Verfahrensschritt I kann aber auch vor dem Verfahrensschritt II erfolgen oder umgekehrt.

Auf Basis der bestimmten Kompressionsdicke d1, d3, d4 und des Ausdehnungsmaßes U1, U2, U3, U4 aus Verfahrensschritt I und Verfahrensschritt II wird daraufhin in einem dritten Verfahrensschritt III die ermittelte Kompressionsdicke d1, d3, d4 und das Ausdehnungsmaß U1, U2, U3, U4 der Brust O1, O2, O3, O4 mittels der Steuereinrichtung erfasst und analysiert. Die Analyse erfolgt dabei bevorzugt mittels Algorithmen, also z. B. mittels einer Look-Up-Tabelle.

In einem weiteren Verfahrensschritt IVb wird so der aktive Bereich der Ultraschallsonde 21 auf Basis des Ausdehnungsmaßes U1, U2, U3, U4 geregelt. In Verfahrensschritt IVa wird auf Basis des ermittelten Ausdehnungsmaßes U1, U2, U3, U4 die Verfahrgeschwindigkeit geregelt. In einem weiteren Verfahrensschritt IVc wird der Verfahrbereich VB1, VB2 der Ultraschallsonde 21 geregelt. In noch einem weiteren Verfahrensschritt IVd wird in Abhängigkeit der ermittelten Kompressionsdicke d1, d3, d4 die Fokuszone der Ultraschallsonde 21 und/oder die Frequenz der ausgesendeten Ultraschallwellen eingestellt.

Für die oben beschriebenen ermittelten Einstellungsparameter, wie beispielsweise den aktiven Bereich, den Verfahrbereich, die Frequenz etc. wird ein Steuerprotokoll erstellt, das die zeitliche Reihenfolge und Durchführung einer Ultraschallaufnahme regelt. Während der Ultraschallaufnahme können so die unter Verfahrensschritt V genannten Parameter adaptiv angepasst werden.

Nach Einstellung der Parameter erfolgt im fünften Verfahrensschritt V die Durchführung eines Ultraschallscans bzw. Ultraschallaufnahme.

So wird z. B. je nachdem auf welcher Position sich die Ultraschallsonde 21 entlang des Verfahrbereichs befindet der aktive Bereich 23 neu eingestellt. Auch wird in Abhängigkeit von dem aktuell eingestellten aktiven Bereich 23 die Verfahrgeschwindigkeit neu angepasst.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. So kann es sich bei der Röntgenanlage nicht nur um ein Mammographiegerät handeln, sondern auch um andere medizinische bildgebende Geräte. Weiterhin schließt die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

### Bezugszeichenliste

1 Röntgenanlage, Mammographieanlage
2 Röntgenquelle
3a, 3b Kompressionsplatten
4 Detektorpixel
5 Röntgendetektor
20 Ultraschallanalage
21 Ultraschallsonde
22 Elemente/ Piezoelemente
23 aktiver Bereich
C1, C2, C3 Cluster
d1, d3, d4 Kompressionsdicke
M Mamille
Oa1, Oa2 Pectoralis
O1, O2, O3, O4 Brust
T1, T2, T3, T4 Ultraschallbildgebungszonen
U1, U2, U3, U4 Ausdehnungsmaß
VB1, VB3, VB4 Verfahrbereich
I, II, III, Iva, IVb, IVc, IVd, V Verfahrensschritte

## Patentansprüche

1. Verfahren zur Steuerung von Ultraschallaufnahmen aufweisend zumindest folgende Schritte:
- Ermittlung einer Kontur (U1, U2, U3, U4) einer Brust (O1, O2, O3, O4) und
- Regelung eines aktiven Bereichs (23) einer Ultraschallsonde (21) einer Ultraschallanlage (20) in Abhängigkeit der ermittelten Kontur (U1, U2, U3, U4).

2. Verfahren nach Anspruch 1, wobei die Kontur (U1, U2, U3, U4) der Brust (O1, O2, O3, O4) mittels einer Aufnahme der Brust(O1, O2, O3, O4) ermittelt wird.

3. Verfahren nach Anspruch 1 oder 2, wobei die Kontur (U1, U2, U3, U4) der Brust (O1, O2, O3, O4) mittels einer Röntgenaufnahme der Brust (O1, O2, O3, O4) ermittelt wird.

4. Verfahren nach einem der vorstehenden Ansprüche, wobei sich der aktive Bereich (23) der Ultraschallsonde (21) bis zu einem Randbereich der Brust (O1, O2, O3, O4) erstreckt.

5. Verfahren nach einem der vorstehenden Ansprüche, wobei der aktive Bereich (23) der Ultraschallsonde (21) durch eine Auswahl an aktiven Elementen (22) der Ultraschallsonde (21) eingestellt wird.

6. Verfahren nach einem der vorstehenden Ansprüche, wobei eine Verfahrgeschwindigkeit der Ultraschallsonde (21) in Abhängigkeit der ermittelten Kontur (U1, U2, U3, U4) geregelt wird.

7. Verfahren nach einem der vorstehenden Ansprüche, wobei eine Dicke (d1, d3, d4) der Brust ermittelt wird und eine Zeit zwischen Aussendung eines Ultraschallsendepuls und Empfang eines Ultraschallempfangspulses auf Basis der ermittelten Dicke (d1, d3, d4) eingestellt wird und gegebenenfalls die Verfahrgeschwindigkeit der Ultraschallsonde (21) angepasst wird.

8. Verfahren nach Anspruch 7, wobei in Abhängigkeit von der ermittelten Dicke (d1, d3, d4) die Frequenz des ausgesendeten Ultraschallsendepulses eingestellt wird.

9. Verfahren nach einem der vorstehenden Ansprüche, wobei in Abhängigkeit der ermittelten Kontur (U1, U2, U3, U4) ein Verfahrbereich (VB1, VB2, VB_{Max}) der Ultraschallsonde (21) eingestellt wird.

10. Verfahren nach einem der vorstehenden Ansprüche, wobei
- mehrere Ultraschallsendepulse bei einer gleichbleibenden Position der Ultraschallsonde (21) ausgesendet werden,
- die durch die mehreren ausgesendeten Ultraschallsendepulse entstehenden Ultraschallbilder zu einem Ultraschallbild zusammengefügt werden und
- sich die Ultraschallsendepulse durch eine unterschiedliche Fokussierung und/oder einer unterschiedlichen Frequenz voneinander unterscheiden.

11. Steuereinrichtung zur Steuerung einer Ultraschallanlage (20), wobei die Steuereinrichtung zumindest
- eine Analyseeinheit zur Bestimmung einer Kontur (U1, U2, U3, U4) einer Brust (O1, O2, O3, O4) und
- eine Regeleinheit zur Regelung eines aktiven Bereichs (23) einer Ultraschallsonde (21) einer Ultraschallanlage (20) in Abhängigkeit der ermittelten Kontur (U1, U2, U3, U4) umfasst.

12. Ultraschallanlage (20) umfassend eine Steuereinrichtung, nach Anspruch 11.

13. Röntgenanlage (1) umfassend eine Ultraschallanlage (20) nach Anspruch 12.

14. Computerprogrammprodukt mit einem Computerprogramm, welches direkt in eine Speichereinrichtung einer Steuereinrichtung einer Ultraschallanlage (20) ladbar ist, mit Programmabschnitten, um alle Schritte des Verfahrens nach einem der Ansprüche 1 bis 10 auszuführen, wenn das Computerprogramm in der Steuereinrichtung der Ultraschallanlage (20) ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 10 auszuführen, wenn die Programmabschnitte von der Rechnereinheit ausgeführt werden.

## Claims

1. Method for controlling ultrasound recordings, including at least the following steps:
- ascertaining a contour (U1, U2, U3, U4) of a breast (O1, O2, O3, O4) and
- regulating an active region (23) of an ultrasound probe (21) of an ultrasound apparatus (20) on the basis of the ascertained contour (U1, U2, U3, U4).

2. Method according to Claim 1, wherein the contour (U1, U2, U3, U4) of the breast (O1, O2, O3, O4) is ascertained by means of a recording of the breast (O1, O2, O3, 04).

3. Method according to Claim 1 or 2, wherein the contour (U1, U2, U3, U4) of the breast (O1, O2, O3, O4) is ascertained by means of an x-ray recording of the breast (O1, O2, O3, 04).

4. Method according to any one of the preceding claims, wherein the active region (23) of the ultrasound probe (21) extends to an edge region of the breast (O1, O2, O3, 04).

5. Method according to any one of the preceding claims, wherein the active region (23) of the ultrasound probe (21) is set by selecting active elements (22) of the ultrasound probe (21) .

6. Method according to any one of the preceding claims, wherein a displacement speed of the ultrasound probe (21) is regulated on the basis of the ascertained contour (U1, U2, U3, U4) .

7. Method according to any one of the preceding claims, wherein a thickness (d1, d3, d4) of the breast is ascertained and the time between emitting an ultrasonic transmission pulse and receiving an ultrasonic reception pulse is set on the basis of the ascertained thickness (d1, d3, d4) and the displacement speed of the ultrasound probe (21) is adapted where necessary.

8. Method according to Claim 7, wherein the frequency of the emitted ultrasonic transmission pulse is set on the basis of the ascertained thickness (d1, d3, d4).

9. Method according to any one of the preceding claims, wherein a displacement range (VB1, VB2, VB_{Max}) of the ultrasound probe (21) is set on the basis of the ascertained contour (U1, U2, U3, U4).

10. Method according to any one of the preceding claims, wherein
- a plurality of ultrasonic transmission pulses are emitted while the position of the ultrasound probe (21) remains unchanged,
- the ultrasound images arising from the plurality of emitted ultrasonic transmission pulses are stitched to form an ultrasound image and
- the ultrasonic transmission pulses differ from one another by way of a different focusing and/or a different frequency.

11. Control device for controlling an ultrasound apparatus (20), wherein the control device comprises at least
- an analysis unit for determining a contour (U1, U2, U3, U4) of a breast (O1, O2, O3, O4) and
- a closed-loop control unit for regulating an active region (23) of an ultrasound probe (21) of an ultrasound apparatus (20) on the basis of the ascertained contour (U1, U2, U3, U4).

12. Ultrasound apparatus (20) comprising a control device according to Claim 11.

13. X-ray apparatus (1) comprising an ultrasound apparatus (20) according to Claim 12.

14. Computer program product with a computer program, which is able to be loaded directly into a memory device of a control device of an ultrasound apparatus (20), comprising program sections for carrying out all steps of the method according to any one of Claims 1 to 10 when the computer program is executed in the control device of the ultrasound apparatus (20).

15. Computer-readable medium, on which program sections which are able to be read and executed by a computer unit are stored in order to carry out all steps of the method according to any one of Claims 1 to 10 when the program sections are executed by the computer unit.

## Revendications

1. Procédé de commande de prises de vue par ultrason, comportant au moins les stades suivants :
- détermination d'un contour (U1, U2, U3, U4) d'un sein (O1, O2, O3, O4) et
- régulation d'une partie (23) active d'une sonde (21) à ultrason d'une installation (20) à ultrason en fonction du contour (U1, U2, U3, U4) déterminé.

2. Procédé suivant la revendication 1, dans lequel on détermine le contour (U1, U2, U3, U4) du sein (O1, O2, O3, O4) au moyen d'une prise de vue du sein (O1, O2, O3, 04).

3. Procédé suivant l'une des revendications 1 ou 2, dans lequel on détermine le contour (U1, U2, U3, U4) du sein (O1, O2, O3, O4) au moyen d'une prise de vue aux rayons X du sein (O1, O2, O3, 04).

4. Procédé suivant l'une des revendications précédentes, dans lequel la partie (23) active de la sonde (21) à ultrason s'étend jusqu'à une partie du bord du sein (O1, O2, O3, 04).

5. Procédé suivant l'une des revendications précédentes, dans lequel on règle la partie (23) active de la sonde (21) à ultrason par un choix d'éléments (22) actifs de la sonde (21) à ultrason.

6. Procédé suivant l'une des revendications précédentes, dans lequel on régule une vitesse de déplacement de la sonde (21) à ultrason en fonction du contour (U1, U2, U3, U4) déterminé.

7. Procédé suivant l'une des revendications précédentes, dans lequel on détermine une épaisseur (d1, d3, d4) du sein et on règle un temps entre l'émission d'une impulsion d'émission d'ultrason et la réception d'une impulsion de réception d'ultrason sur la base de l'épaisseur (d1, d3, d4) déterminée et, le cas échéant, on adapte la vitesse de déplacement de la sonde (21) à ultrason.

8. Procédé suivant la revendication 7, dans lequel on règle la fréquence de l'impulsion d'émission d'ultrason émise en fonction de l'épaisseur (d1, d3, d4) déterminée.

9. Procédé suivant l'une des revendications précédentes, dans lequel on règle une partie (VB1, VB2, VB_{Max}) du déplacement de la sonde (21) à ultrason en fonction du contour (U1, U2, U3, U4) déterminé.

10. Procédé suivant l'une des revendications précédentes, dans lequel
- on émet plusieurs impulsions d'émission d'ultrason pour une position restant la même de la sonde (21) à ultrason,
- on assemble les images d'ultrason créées par les plusieurs impulsions d'émission d'ultrason émises en une image d'ultrason et
- on distingue les unes des autres les fréquences d'émission d'ultrason par une focalisation différente et/ou par une fréquence différente.

11. Dispositif de commande pour la commande d'une installation (20) à ultrason dans lequel le dispositif de commande comprend au moins
- une unité d'analyse pour la détermination d'un contour (U1, U2, U3, U4) d'un sein (O1, O2, O3, O4) et
- une unité de régulation pour réguler une partie (23) active d'une sonde (21) à ultrason d'une installation (20) à ultrason en fonction du contour (U1, U2, U3, U4) déterminé.

12. Installation (20) à ultrason, comprenant un dispositif de commande suivant la revendication 11.

13. Installation (1) à rayons X, comprenant une installation (20) à ultrason suivant la revendication 12.

14. Produit de programme d'ordinateur, ayant un programme d'ordinateur, qui peut être chargé directement dans un dispositif de mémoire d'un dispositif de commande d'une installation (20) à ultrason et ayant des parties de programme pour effectuer tous les stades du procédé suivant les revendications 1 à 10 lorsque le programme d'ordinateur est réalisé dans le dispositif de commande de l'installation (20) à ultrason.

15. Support déchiffrable par ordinateur, sur lequel sont mises en mémoire des parties de programme déchiffrables et réalisables par une unité informatique pour effectuer tous les stades du procédé suivant l'une des revendications 1 à 10 lorsque les parties de programme sont réalisées par l'unité informatique.
